# EUROPEAN PATENT APPLICATION

(11) **EP 2 008 588 A1**
(43) Date of publication of application: **31.12.2008**
(21) Application number: 07741804.4
(22) Date of filing: 17.04.2007
(51) Int. Cl.: A61B 5/151

(54) **BIOSENSOR CHIP**

(30) Priority: 17.04.2006 JP 2006113915
(71) Applicant: Sumitomo Electric Industries, Ltd., Osaka-shi, Osaka 541-0041 (JP); National Institute of Advanced Industrial Science and Technology, Tokyo 100-8921 (JP)
(72) Inventor: KITAMURA, Takahiko, Osaka-shi, Osaka 554-0024 (JP); KAIMORI, Shingo, Osaka-shi,Osaka 554-0024 (JP); HARADA, Akira, Osaka-shi,Osaka 554-0024 (JP); HOSOYA, Toshifumi, Osaka-shiOsaka 554-0024 (JP); FUJIMURA, Tsuyoshi, Tsukuba-shi, Ibaraki 305-8562 (JP); KARUBE, Isao, Tsukuba-shi, Ibaraki 305-8562 (JP); GOTOH, Masao, Tsukuba-shi, Ibaraki 305-8562 (JP); NAKAMURA, Hideaki, Tsukuba-shi, Ibaraki 305-8562 (JP); ISHIKAWA, Tomoko, Tsukuba-shi, Ibaraki 305-8562 (JP)
(74) Representative: Cross, Rupert Edward Blount
(86) International application number: PCT/JP2007/058368
(87) International publication number: WO 2007/119853

(57) **Abstract**

There is provided a biosensor chip where the collecting amount necessary for the measurement is made small whereby burden of a user is reduced and, at the same time, a sample at a puncture opening is able to be easily collected and measured without an operation of making the sample collection opening nearer the puncture opening.

When an end 11a of a main body chip 11 is pushed onto a test body M, an elastic body 20 installed at an end 11a of the main body chip 11 is compressed and a device for puncture 12 is produced whereby the test body M is able to be punctured.

When the pushing force is made weak, the device for puncture 12 is pulled out from the test body M due to resilience of the elastic body 20 and a sample D is flown out from the puncture opening. At that time, since the puncture opening and the sample collection opening 13 installed in the main body chip 11 are enclosed in a tightly-closed space 23 formed by the elastic body 20, even small amount of the sample D is able to be easily collected by the sample collection opening 13 without conducting the positioning of the sample collection opening 13.

## Description

### Technical Field

The present invention relates to a biosensor chip and, for example, it relates to a biosensor chip which conducts measurement and analysis of chemical substances using a reagent received in a hollow reacting part of the chip.

### Related Art

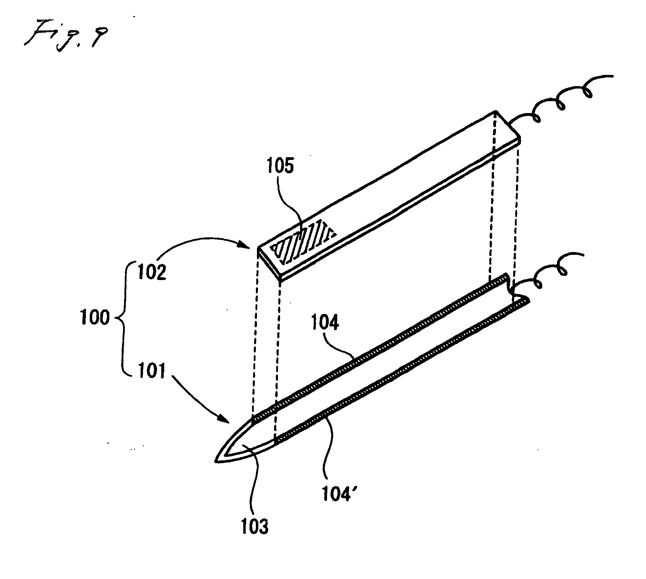

There has been already known a biosensor chip which detects, for example, the concentration of glucose in blood (refer, for example, to Patent Document 1).
Fig. 9 is a disassembled oblique view showing the glucose sensor mentioned in Patent Document 1.
As shown in Fig. 9, the glucose sensor 100 which is a biosensor has a pair electrode 101 and a working electrode 102. The pair electrode 101 has a hollow needle shape which is cut into half in the longitudinal direction and its front end 103 is obliquely cut in a needle shape so as to easily puncture. The surface cut into half is applied with insulating layers 104, 104' which also act as adhesive layers such as an epoxy resin adhesive, a silicone adhesive or glass and the working electrode 102 is installed via the insulating layers 104, 104'. The working electrode 102 is a material in a flat plate shape where glucose oxidase (GOD) is fixed and is adhered to the pair electrode 101 where the side in which GOD is fixed is turned inside. Accordingly, the front end 103 of the needle-shaped pair electrode 101 is punctured to a person to be tested to collect blood and the reaction of the collected blood with the fixed GOD 105 is detected by the working electrode 102 whereupon a quantitative determination of glucose is carried out.

There is also disclosed a biosensor in which biosensor chip and lancet are integrated (refer, for example, to Patent Document 2).
Fig. 10(A) is an oblique view of the sensor mentioned in Patent Document 2 and Fig. 10 (B) is a disassembled oblique view of the sensor. As shown in Fig. 10, a sensor 110 integrated with a lancet has a chip main body 111, a lancet 113 and a protective cover 115. The chip main body 111 has a cover 111a and a substrate 111b in a freely openable manner and an internal space 112 is formed in the inner side of the cover 111a. The internal space 112 has such a shape which is able to receive the lancet 113 in a movable manner.

A needle installed at the front end of the lancet 113 is able to come in and out at an opening 112a formed at the front end of the internal space 112 of the chip main body 111 as the lancet 113 moves. The shape of the internal space 111a is curved at the end where the projection 113a is positioned so that its width becomes somewhat narrower than the lancet 113 and the lancet 113 is locked by the chip main body 111 by the pushing force and the abrasive force of them. A protective cover 115 has a pipe 115a into which a needle 114 is inserted and, as the needle 114 moves, the pipe 115a is also able to be received in the inner area of the chip main body 111. Accordingly, in the state before actual use, the protective cover 115 is covered on the needle 114 so that it protects the needle 114 and does not erroneously injure a user. In the substrate 111b, a pair of electrode terminals 116 is installed so that it is able to be electrically connected to a measuring device (not shown).

In use, the protective cover 115 is detached and the lancet 113 is pushed so that the needle 114 is projected out from the chip main body 111. A sample body is punctured under this state, then the needle 114 is received in the inner area of the chip main body 111, an opening 112a installed at the front end of the chip main body 111 is brought nearer to the puncture opening and the blood flown out therefrom is collected.
[Patent Document 1] JP-A-2-120655
[Patent Document 2] Pamphlet of International Publication WO 02/056,769

### Disclosure of the Invention

### Problems to be solved by the Invention

However, since a needle-shaped pair of electrodes 101 and a working electrode 102 are formed by adhesion in the glucose sensor mentioned in Patent Document 1, diameter of the puncture needle becomes as big as in the same size as the width of the glucose sensor 100. Therefore, there are problems that the amount of the collecting blood becomes too much and pain upon puncture is high whereby the burden of the user becomes big.
Moreover, in a sensor 110 which is integrated with a lancet as mentioned in Patent Document 2, it has a structure where the blood flown out from the puncture opening is absorbed from an opening 112a and such a structure is complicated.

The present invention is achieved in view of the above-mentioned problems and its object is to provide a biosensor in which the collecting amount of a sample necessary for the measurement is made small so that burden of a user is reduced and, at the same time, a sample at the puncture opening is able to be collected and measured without conducting an operation of bringing the sample collection opening nearer to the puncture opening.

### Means for solving the Problems

In order to achieve the above-mentioned object, according to a first aspect of the invention, there is provided a biosensor chip including:
a chip main body,
electrodes for detection,
a device for puncture fixed to an end of the chip main body, and
an elastic body installed on the end of the chip main body.
In the present invention, a device for puncture covers needle, lancet needle, cannula, etc.

When an end of the chip main body is pushed to a test body in the biosensor chip constituted as such, an elastic body installed at an end of the chip main body is compressed and a device for puncture is projected whereby the test body is able to be punctured. When a pushing pressure made weak, the device for puncture is pulled out from the test sample due to resilience of the elastic body whereupon the sample is flown out from the puncture opening. Incidentally, when puncture is carried out in case the inner area of the protective cap covering the chip main body is in a reduced pressure, a sample is efficiently flown out therefrom.

According to a second aspect of the invention, there is provided the biosensor chip according to the first aspect, wherein
the elastic body is provided with a space by which the device for puncture is capable to suck a sample after the puncture.

In the biosensor chip constituted as such, suction of the sample after the puncture is able to be conducted via a space of the elastic body whereby it is now possible to suck the sample smoothly even when the amount of the sample is small.

According to a third aspect of the invention, there is provided the biosensor chip according to the first or second aspect, wherein
a sample collection opening formed at a front end of the chip main body and a puncture opening formed on a test body by the device for puncture are connected by a tightly-closing and semi-opening space.

In the biosensor chip constituted as such, a puncture opening and a sample collection opening installed at the front end of a chip main body are connected by a tightly-closing and semi-opening space formed by an elastic body whereby, upon the puncture, even small amount of the sample is able to be easily collected by the sample collection opening. Incidentally, when the device for puncture is not made projected out form an end of the elastic body before use, protection of the device for puncture and protection of users are able to be achieved. Further, when the device for puncture is not made projected out from an end of the elastic body in disposal after use, it is also possible to dispose safely and appropriately.

According to a forth aspect of the invention, there is provided the biosensor chip according to any one of the first to third aspects, wherein
the device for puncture is projected at the end.

In the biosensor chip constituted as above, there is a characteristic feature that, since the device for puncture is projected at one end, the sample is able to be easily collected.

According to a fifth aspect of the invention, there is provided the biosensor chip according to any one of the first to forth aspects, further including:
a driving mechanism which punctures the device for puncture into a test body.

In the biosensor chip constituted as such, time for the puncture is able to be made short by puncturing the device for puncture into a test body by means of a driving mechanism, it is now possible to reduce the pain upon collecting the sample.

According to a sixth aspect of the invention, there is provided a method for collecting a sample, including the steps of:
preparing the biosensor chip according to any one of the first to the fifth aspects,
puncturing the elastic body by means of compression by pushing to the test body,
collecting a sample with forming a flow path, and
pulling the device for puncture out of the test body by resilience of the elastic body.

In the sample collection method constituted as such, the elastic body installed at an end of the biosensor chip is pushed onto the test body, the elastic body is compressed and the device for puncture is projected from the front end of the elastic body whereby the device for puncture is pulled out by resilience of the elastic body after the puncture. As a result, analysis using a small amount of the sample is possible whereby burden of the test body is able to be reduced.

According to a seventh aspect of the invention, there is provided a biosensor system including:
the biosensor chip according to any one of the first to the fifth aspects, and
a measuring device receiving information of collected sample by connecting to the electrodes for detection of the biosensor chip.

In the biosensor system constituted as such, a sample is collected by the above-mentioned biosensor chip and information of the sample is transmitted to a measuring device via the detecting electrodes whereby the measurement is able to be conducted easily within short time and, as a result, burden of a test body is able to be reduced.

### Effects of the Invention

In accordance with the present invention, an elastic body is installed at an end of the chip main body and, as a result, when an end of the chip main body is pushed onto a test body, the elastic body installed at an end is compressed and the device for puncture is projected whereby it is able to puncture the test body. When the pushing force is made weak, the device for puncture is pulled out from the test body due to resilience of the elastic body whereby a sample is flown out from the puncture opening. Since a puncture opening and the sample collection opening installed at the front end of the chip main body are enclosed in a tightly-closing and semi-opening space formed by the elastic body whereby even a small amount of the sample is able to be easily collected by the sample collection opening and burden of a test body is able to be reduced.

### Brief Description of Drawings

Fig. 1(A) is an explanatory drawing which shows an embodiment of the biosensor chip according to the present invention, and Fig. 1 (B) is an explanatory drawing which shows an embodiment of the biosensor chip according to the present invention.
Fig. 2 is a plane figure which shows an embodiment of the biosensor system according to the present invention.
Figs. 3(A) to (C) are explanatory drawings which show an operation of measurement of blood sugar level using the biosensor system according to the present invention.
Fig. 4 (A) is an explanatory drawing which shows another embodiment of the biosensor chip according to the present invention, and Fig. 4 (B) is an explanatory drawing which shows another embodiment of the biosensor chip according to the present invention.
Fig. 5(A) is a drawing which shows another embodiment of the elastic body used in the biosensor chip according to the present invention, and Fig. 5 (B) is a drawing which shows another embodiment of the elastic body used in the biosensor chip according to the present invention.
Fig. 6 (A) is a drawing which shows still another embodiment of the elastic body used in the biosensor chip according to the present invention, and Fig. 6 (B) is a drawing which shows a modified example of Fig. 6(A).
Fig. 7 (A) is a drawing which shows still another embodiment of the elastic body used in the biosensor chip according to the present invention, and Fig. 7 (B) is a drawing which shows another embodiment of the elastic body used in the biosensor chip according to the present invention.
Fig. 8 is a drawing which shows still another embodiment of the elastic body used in the biosensor chip according to the present invention.
Fig. 9 is a disassembled oblique view which shows the conventional biosensor chip.

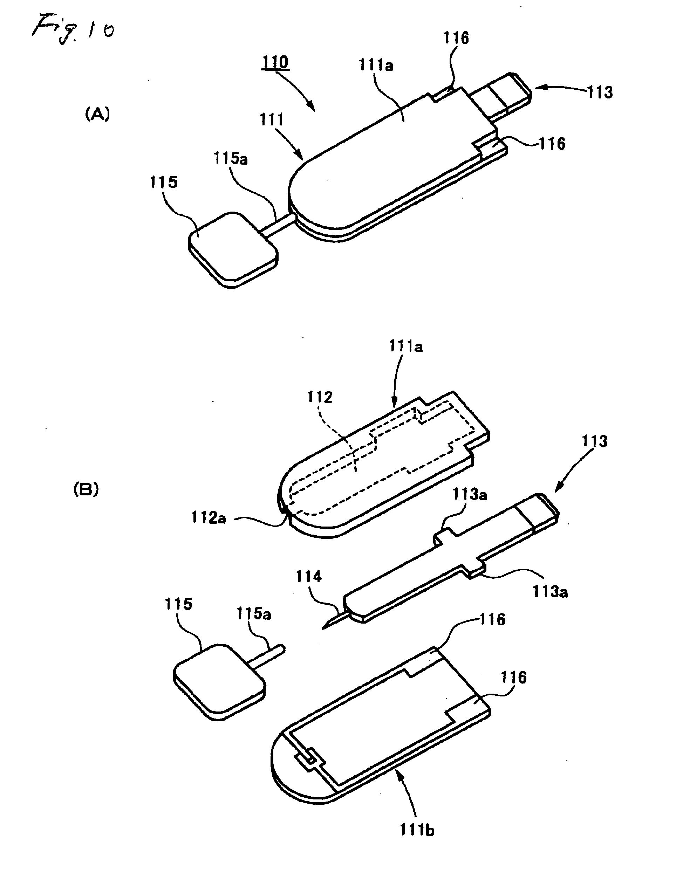

Fig. 10(A) is an oblique view which shows the conventional biosensor chip, and Fig. 10(B) is a disassembled oblique view which shows the conventional biosensor chip.

### Description of Referential Numerals

- 10: biosensor chip
- 11: main body chip
- 11a: an end
- 12: device for puncture
- 12a: front end
- 13: sample collection opening
- 14: reagent
- 18a,: 18b electrodes for detection
- 20: elastic body
- 21: front end (surface)
- 23: tightly-closed space
- 24: adhesive
- 30: biosensor system
- 31: measuring device
- D: blood (sample)
- M: test body

### Best Mode for Carrying Out the Invention

As hereunder, embodiments of the present invention will be illustrated in detail by referring to the drawings.

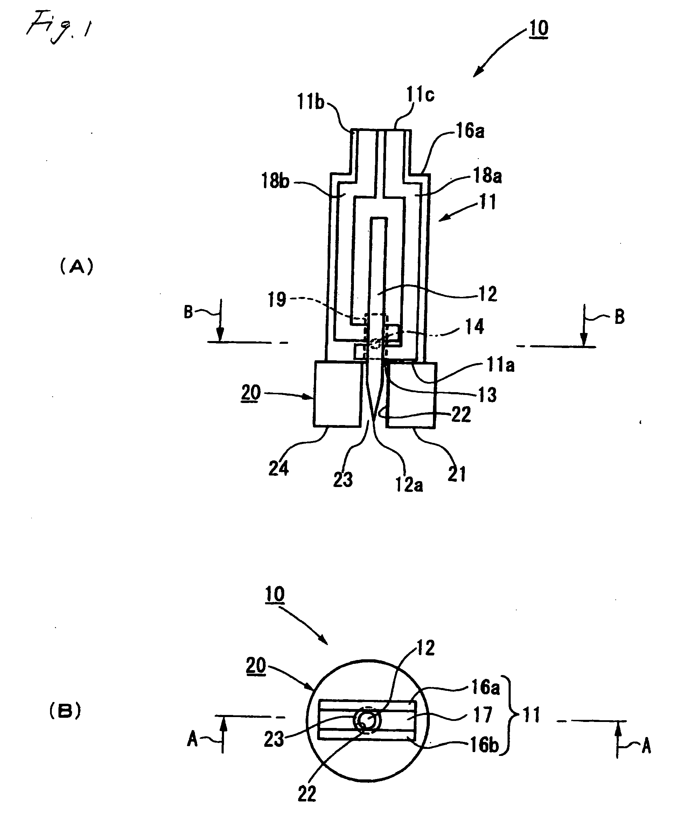

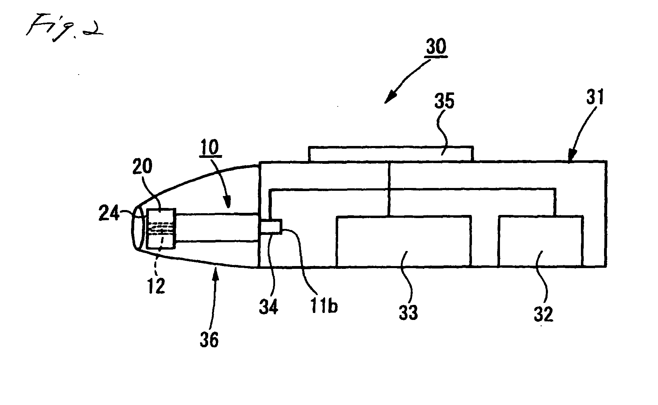

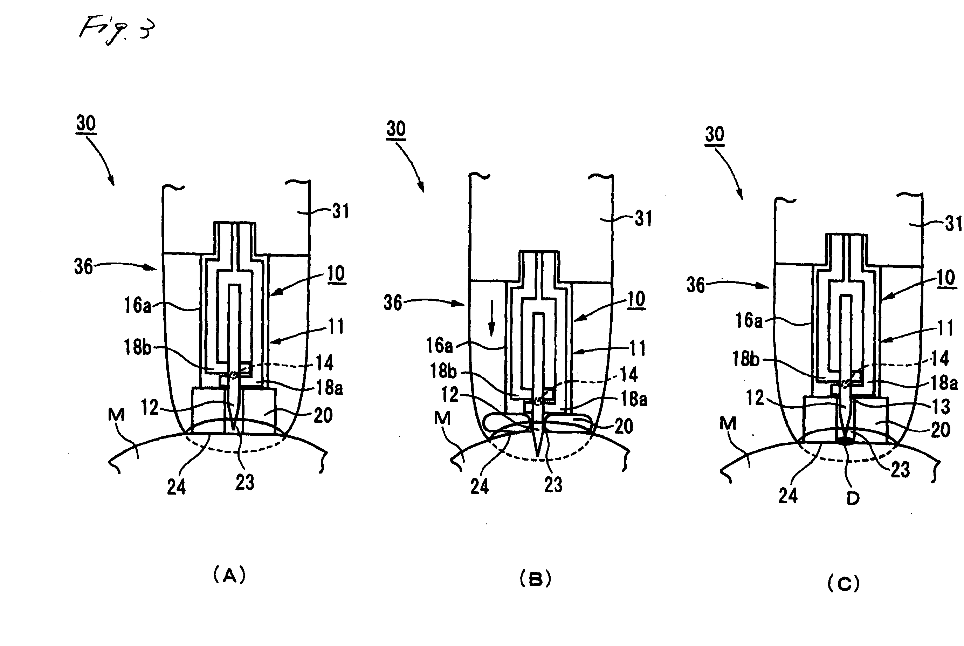

Fig. 1 (A) is a cross-sectional view of the position A-A in Fig. 1 (B) which shows an embodiment of the biosensor chip of the present invention; Fig. 1 (B) is a cross-sectional view of the position B-B in Fig. 1 (A) which shows an embodiment of the biosensor chip of the present invention; Fig. 2 is a constitutional drawing which shows an embodiment of the biosensor system of the present invention; and Fig. 3(A) to (C) are explanatory drawings which show a collecting operation of a sample using the biosensor system according to the present invention.

As shown in Fig. 1(A) and (B), the biosensor chip 10 which is an embodiment of the present invention has a chip main body 11 and a device for puncture 12 for the puncture which is fixed to an end 11a of the chip main body 11 where a front end thereof 12a is projected. An elastic body 20 which forms, upon pushing onto a test body M, a sample collection opening 13 installed at an end 11a of the chip main body 11 and a tightly-closing and semi-opening space 23 by enclosing a puncture opening formed on the test body M by a device for puncture 12 is installed at an end 11a of the chip main body 11.

The tightly-closing and semi-opening space 23 becomes a tightly-closed one when an elastic body 20 is pushed onto a test body whereby the sample is able to be easily collected.

The chip main body 11 has two substrate plates 16a, 16b facing each other and a spacer layer 17 being sandwiched between the two substrate plates 16a, 16b. Electrodes for detection 16a, 16b are installed on the surface of the spacer layer 17 side of at least one substrate plate 16a of the two substrate plates 16a, 16b and their front ends (the lower ends in Fig. 1(A)) are bent in an L-shape in the direction of being faced each other whereby a predetermined space is kept. A hollow reacting area 19 is formed by the two substrate plates 16a, 16b and the spacer layer 17 throughout the area ranging from an end 11a of the chip main body 11 to the part where two electrodes for detection 18a, 18b are faced each other. At the front end of the hollow reacting area 19, there is installed a sample collection opening 13 by which the blood D (refer to Fig. 3(C)) is collected as a sample by the device for puncture 12 by means of puncturing into a test body M (refer to Fig. 3) and introduced into a hollow reaction area 19.

Thus, both upper and lowers surfaces of the hollow reacting area 19 are formed by the substrate plates 16a, 16b and the electrodes for detection 18a, 18 and a rectangular space is formed using a spacer 17 which is cut into a predetermined shape as a side wall. Therefore, the electrodes for detection 18a, 18b are exposed in the hollow reacting area 19 and, immediately above or near the electrodes for detection 18a, 18b in the hollow reacting area 19, there is installed a reagent 14 which, for example, fixes an enzyme and a mediator and generates electric current by the reaction with glucose in the blood D. Accordingly, the hollow reacting area 19 is an area where the blood D such as blood taken thereinto from a sample collection opening 13 is subjected to biochemical reaction with the reagent 14.

With regard to an elastic body 20 installed at an end 11a of a chip main body 11, there may be exemplified a cylindrical one having a perforation hole 22 for forming a tightly-closing and semi-opening space 23 at the center. Since a device for puncture 12 is inserted into the perforation hole 22, diameter of the hole is larger than the outer diameter of the device for puncture 12. Thickness of an elastic body 20 is sufficiently thick so that it is able to surely cover until the front end of the device for puncture 12. As to a material for the elastic body 20, there is no particular limitation so far as it is elastic and examples of the applicable one are rubber or sponge including a single polymer of silicone, urethane, acrylate, ethylene, styrene, etc. or a copolymer thereof; polyolefin such as polyethylene and polypropylene; polyester such as polyethylene terephthalate and polybutylene terephthalate; and fluorine resin such as polytetrafluoroethylene and PFA which is a copolymer of perfluoroalkoxyethylene with polyfluoroethylene. The rubber elastomer may be hollow or not.

It is desirable that the front end 21 which is a surface of the elastic body contacting the test body M is constituted from a material such as adhesive silicone rubber, acrylate rubber, etc. or the elastic body 20 is mixed with or coated with an adhesive 24.
It is not essential that the front end 21 has adhesive property and even a material having no adhesive property is able to be substituted for an adhesive by installing very fine projections for preventing slippage. As a result, it is now possible that close adhesion of the elastic body 20 with the test body M is improved, that slippage from the puncture position is prevented and that a tightly closed space 23 is surely formed.

Examples of a driving mechanism for puncturing a device for puncture into a test body are spring and motor. When such a driving mechanism is used, time needed for the puncture is able to be made short and the pain upon the puncture is able to be reduced.

Now the biosensor system according to the present invention will be illustrated. Fig. 2 shows a constitution of a biosensor system 30 using the above-mentioned biosensor chip 10.
As shown in Fig. 2, the biosensor system 30 has the above-mentioned biosensor chip 10, a measuring device 31 which connects to electrodes for detection 18a, 18b of the biosensor chip 10 and obtains the information of blood D collected by connecting and a protective cap 36 for the biosensor chip. Constitution of the biosensor chip 10 is as mentioned above and the sites which are common to those in the already-mentioned biosensor chip 10 are assigned with the same symbols whereby illustrations thereof will be omitted here.

The measuring device 31 is equipped with an electric source 32, a controlling device 33, a terminal insertion area 34 and a display area 35 and they are connected each other.
The terminal insertion area 34 is fixed by being inserted with the rear end 11b of the chip main body 11 of the biosensor chip 10 and the electrodes for detection 18a, 18b exposed to the rear end 11c of the chip main body 11 are electrically connected. This biosensor system 30 is small in size and is a handy type which is, for example, able to be held by one hand of a test body.

Now the method of use will be illustrated by taking the case where sugar blood level is measured using this biosensor system 30 by referring to Fig. 3(A) to (C).
Firstly, the rear end 11b of the main body 11 of a biosensor chip 10 is inserted into a terminal insertion part 34 of a measuring device 31 to fix and to electrically connect as shown in Fig. 2. The electric source 32 of the biosensor system 30 is made on and it is confirmed whether the system is driven normally. As shown in Fig. 3 (A), the biosensor system 30 is held and the protective cap 36 is pushed onto a test body so that the punctured area is congested with blood and the elastic body 20 attached to an end 11a of the biosensor chip 10 is contacted to the blood collection area of the test body M. Since the front end of the elastic body 20 is coated with an adhesive 24, getting-out of the position during the operation thereafter is able to be prevented.

After that, the biosensor chip 10 is pushed onto the test body M as shown in Fig. 3(B). As a result, the elastic body 20 is crushed and a device for puncture 12 is projected from the front end of the elastic body 20 and punctures the test body M.

When the force of pushing the biosensor chip 20 is made weak as shown in Fig. 3(C), the elastic body 20 returns to its original state (the state of Fig. 3 (A)) due to the resilience whereby the device for puncture 12 is detached from the test body M. At that time, inner area of the tightly-closed space including the puncture opening becomes negative pressure and, therefore, blood D is apt to be flown out from the puncture opening. Further, since the inner surface of the penetration hole 22 forming the tightly-closed space 23 is subjected to a hydrophilic treatment, the blood D is collected from the sample collection opening 13 along the inner surface of the penetration hole 22 due to its surface tension and capillary phenomenon. The collected blood D is introduced into a hollow reaction area 19. At that time, the sample collection opening 13 is positioned in the tightly-closed space 23 together with the puncture opening formed by the device for puncture 12 and, therefore, the blood D is able to be collected easily and surely without moving the biosensor chip 10. As a result, even a test body M with poor eyesight is able to use it and, further, the measurement is possible using small amount of blood whereupon burden of the test body upon collection of the blood is able to be reduced. In addition, since the tightly-closing and semi-opening space 23 is shut out from the air outside, coagulation of the blood D is retarded whereby its collection is able to be made easier.

When a predetermined amount of blood is collected, the biosensor system 30 is detached from the test body M and one waits until the measured result is shown on a display 35. The blood D which is introduced into the hollow reacting part 19 reacts with the reagent 14 and the data of electric current or electric charge (charge amount) measured by the electrodes for detection 18a, 18b are sent to a controlling device 33. A calibration curve data table is received in the controlling device 33 and then the calculation of blood sugar level is conducted on the basis of the measured electric current value (charge amount). When the calculation finishes, the measured result is shown on a display 35 and the sugar blood level, for example, is able to be expressed in numerals. Finally, the biosensor chip 10 is detached from the measuring device 31 and, since the elastic body 20 returns at that time almost to the original height, the state where the device for puncture 12 is not projected from an end 11a of the chip main body 11 is achieved. As a result, a user is able to appropriately treat the biosensor chip 10 after use without being injured by the device for puncture 12.

When the burden of the test body for the collection of blood is taken into consideration, volume of the hollow reaction part 19 is preferably not more than 1 µL (microliter) and, particularly preferably, not more than 300 nL (nanoliter). When the hollow reacting part 19 is as small as such, a sufficient amount of blood of the test body is able to be collected even when the diameter of the device for puncture 12 is small. Preferably, the diameter is not more than 1,000 µm.

When an end 11a of the chip main body 11 is pushed onto a test body M in the above-mentioned biosensor chip 10 and biosensor system 30, the elastic body 20 is compressed and the device for puncture 12 is projected whereupon the test body M is able to be punctured. When the pushing force is made weak, the device for puncture 12 is pulled out from the test body M due to the resilience of the elastic body 20 whereupon the blood D is flown out from the puncture opening. At that time, the puncture opening and the sample collection opening 13 installed at an end 11a of the chip main body 11 are enclosed in a tightly-closed space 23 formed by the elastic body 20 and, when the elastic body 20 returns to its original shape after the puncture, the inner area of the tightly-closed space 23 becomes a negative pressure whereby the blood D is able to be collected from a very small puncture opening and pain of the test body M is able to be reduced. In addition, even small amount of blood D is able to be easily collected by the sample collection opening and analyzed and, therefore, burden of the test body M is able to be reduced.
Further, when the device for puncture 12 is made not to project from the front end 21 of the elastic body 20 before the use, protection of the device for puncture 12 and protection of users are able to be achieved. Furthermore, when the device for puncture 12 is made not to project from the front end 21 of the elastic body 20 upon disposal after the use, it is possible to dispose safely and adequately.

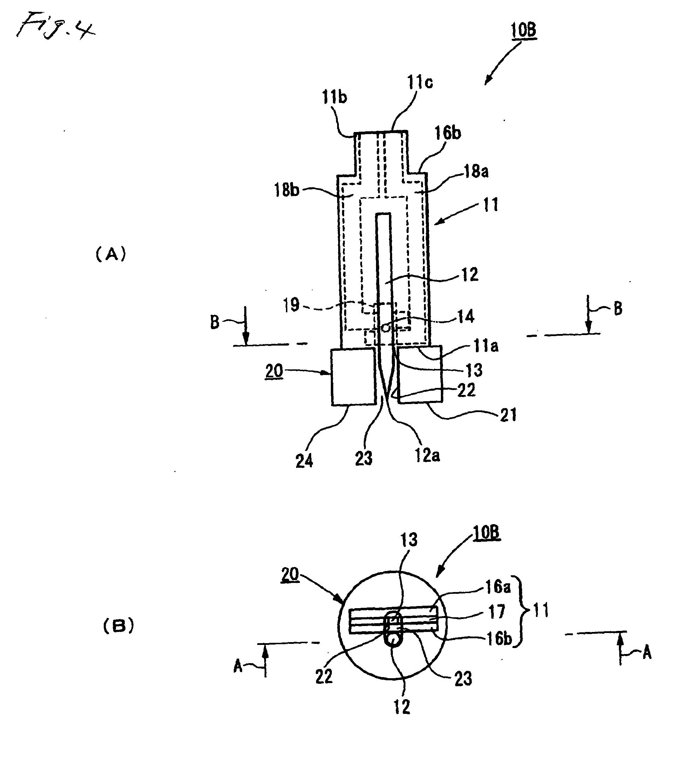

The biosensor chip of the present invention is not limited to the above-mentioned embodiments only but may be appropriately modified, improved, etc.
For example, in the above-mentioned embodiment, an example is shown where the device for puncture 12 is installed in a spacer layer 17 being sandwiched between both of the substrate plates 16a, 16b but the biosensor chip 10 of the present invention is not limited thereto. For example, it is also possible to install a device for puncture 12 along the outer surface of one of the substrate plates 16a as shown in Figs. 4 (A) and (B) . In the case of the biosensor chip 10B as such, it is possible to reduce the thickness of the chip main body 11 whereby a thin biosensor chip 10B is able to be formed. However, since the device for puncture 12 and the sample collection opening 13 are somewhat apart, it is desirable that a cross-sectional shape of the penetration hole 22 is made rectangular or the like and the gap formed between the outer surface of the device for puncture 12 and the inner surface of the penetration hole 22 of the elastic body 20 is made as small as possible. Incidentally, in Fig. 4, the sites which are common to those in the already-mentioned biosensor chip 10 are assigned with the same numerals whereby the duplicated illustrations are able to be omitted.

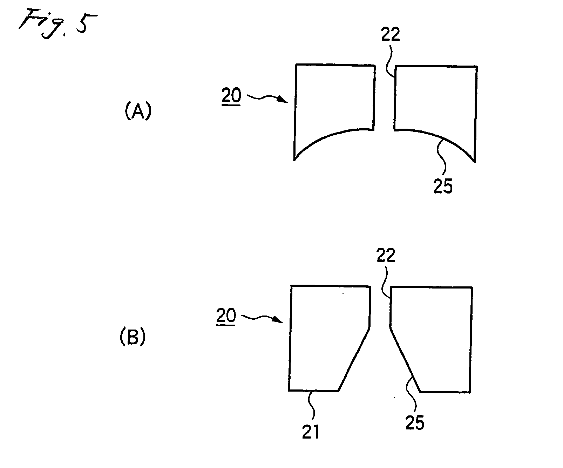

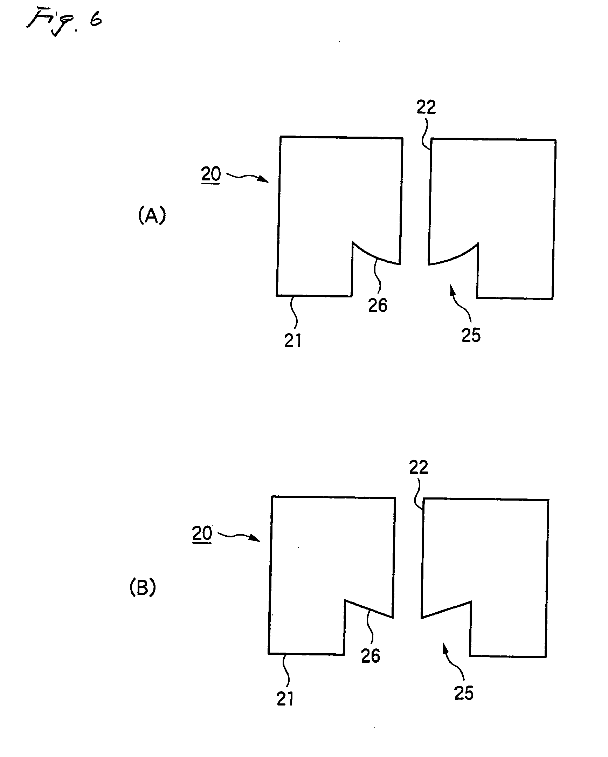

It is also possible that, as shown in Fig. 5, the shape of the part of the elastic body 20 to be pushed onto the test body is made into a curved or linear hollow 25 so as to enhance the adhesive property to fingers. It is further possible that, as shown in Fig. 6, a columnar hollow 25 is formed around the penetration hole 22 of the front end 21 of the elastic body 20 so as to enhance the adhesive property to fingers. Thus, it is now possible to efficiently collect the blood by forming a hollow (gap) between the penetration hole which is a blood collection opening of the elastic body and the fingers. That is particularly highly effective when the collecting amount is small.

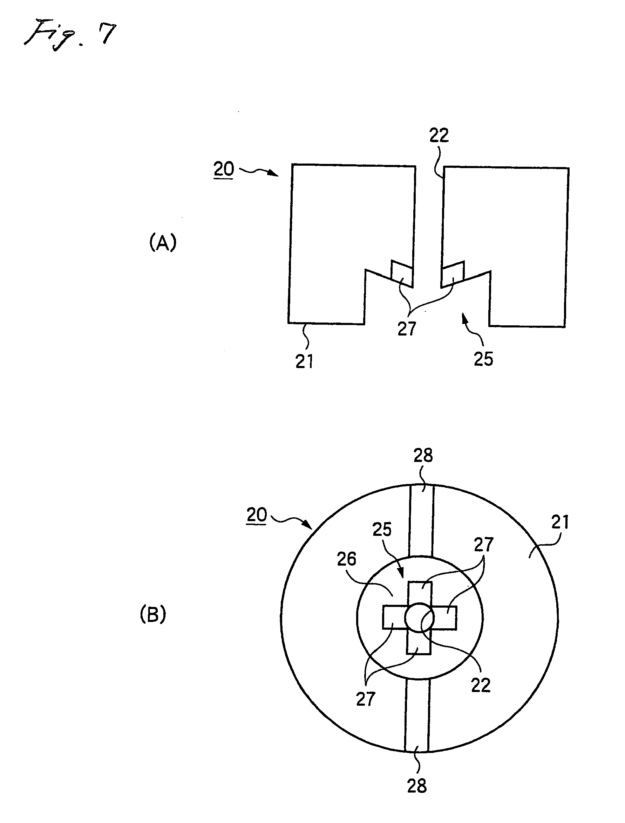

Still another embodiment of the elastic body is that, as shown in Fig. 7, plural cuttings 27 are formed at the linear projected area 26 surrounding the penetration hole 22 of the front end 21 of the elastic body 20 where the central part of the columnar hollow 25 is in a front end direction and the cut grooves 28 are formed on the front end surface 21 whereby the resilience of the front end 21 and the projection 26 is able to be enhanced.

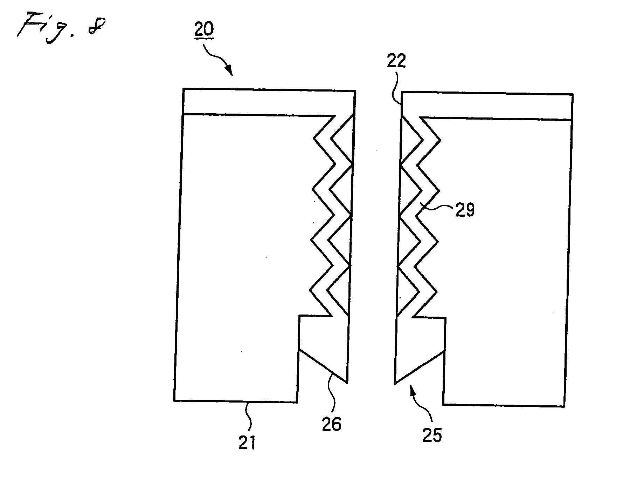

A furthermore embodiment of the elastic body is that, as shown in Fig. 8, the elastic body 20 is not necessary to be integrally formed by a single type of a material but an elastic body having a function of pulling out the device for puncture 12 from the test body and a path for introducing the blood may be formed from different materials. Thus, it is also possible that, as to the area which directly contacts the blood, an expansion part 29 is formed by molding a material such as fluorine resin used for artificial blood vessel, etc. into a pleated shape, a projection 26 is formed at the front end and then they are integrally molded with the elastic body. It is still further possible that the biosensor chip and the rubber elastomer are engaged each other so that the fixation is made stronger.

In the above-mentioned embodiments, illustration is made for the cases where the test body M or the like is punctured by pushing the biosensor chips 10, 10B but an embodiment where the puncture is carried out by a lancet is also possible.
Further, although the cases where collection is carried out by means of surface tension or capillary phenomenon of the blood D are illustrated hereinabove, it is furthermore possible to use a device such as a pump which sucks the blood D which is flown out to a puncture opening. It is still further possible that sucking is conducted when a tightly-closed space 23 is made in reduced pressure.

Hereinabove, the present invention is illustrated in detail and also by referring to specific embodiments but it is apparent for persons skilled in the art that various changes and modifications thereof are able to apply without departing from the spirit and the scope of the present invention.
The present invention is based on the Japanese Patent Application (No. 2006-113, 915) filed on April 17, 2006 and its content is incorporated herein as a reference.

### [Industrial Applicability]

As mentioned hereinabove, an elastic body is installed at an end of a chip main body in the biosensor chip of the present invention and, therefore, the elastic body installed at an end of the chip main body is compressed and a device for puncture is projected whereby a test body is punctured. When the pushing force is made weak, the device for puncture is pulled out from the test body due to the resilience of the elastic body whereupon a sample is flown out from the puncture opening. At that time, since the puncture opening and the sample collection opening installed at an end of the chip main body are enclosed in a tightly-closed space formed by the elastic body, there is achieved an effect that even small amount of sample is able to be easily collected by the sample collection opening and it is useful as a biosensor chip, etc. which conducts the measurement and the analysis of chemical substances using a reagent received in a hollow reaction part of the chip.

## Claims

1. A biosensor chip comprising:
a chip main body,
electrodes for detection,
a device for puncture fixed to an end of the chip main body, and
an elastic body installed at the end of the chip main body.

2. The biosensor chip according to claim 1, wherein
the elastic body is provided with a space by which the device for puncture is capable to suck a sample after the puncture.

3. The biosensor chip according to claim 1 or 2, wherein
a sample collection opening formed at a front end of the chip main body and a puncture opening formed on a test body by the device for puncture are connected by a tightly-closing and semi-opening space.

4. The biosensor chip according to any one of claims 1 to 3, wherein
the device for puncture is projected at the end.

5. The biosensor chip according to any one of claims 1 to 4, further comprising:
a driving mechanism which punctures the device for puncture into a test body.

6. A method for collecting a sample, comprising the steps of:
preparing the biosensor chip according to any one of claims 1 to 5,
puncturing the elastic body by means of compression by pushing to the test body,
collecting a sample with forming a flow path, and
pulling the device for puncture out of the test body by resilience of the elastic body.

7. A biosensor system comprising:
the biosensor chip according to any one of claims 1 to 5, and
a measuring device receiving information of collected sample by connecting to electrodes for detection of the biosensor chip.
